# EUROPEAN PATENT SPECIFICATION

(11) **EP 0 072 556 B1**
(45) Date of publication and mention of the grant of the patent: **29.01.1986**
(21) Application number: 82107399.6
(22) Date of filing: 13.08.1982
(51) Int. Cl.: A01N 31/14, A01N 37/02, C07C 43/215, C07C 43/23, C07C 69/145, C07C 41/16, C07C 41/26, C07C 41/30

(54) **Acaricide compounds**
Acarizide Verbindungen
Composés acaricides

(30) Priority: 14.08.1981 IT 2351881
(43) Date of publication of application: 23.02.1983
(73) Proprietor: Montedison S.p.A., I-20121 Milan (IT)
(72) Inventor: Bettarini, Franco, Novara (IT); Regiroli, Giovanni, Novate Milanese (Milano) (IT); Massardo, Pietro, Milano (IT)
(74) Representative: Weinhold, Peter, Dr.

## Description

This invention relates to new acaricide compounds and, more particularly, to new diethers of 4-hydroxy-benzyl alcohol, the processes for preparing said diethers, the use thereof as acaricides and acaricide compositions containing them.

Several compounds endowed with an acaricide activity are known, some of which being active against the mites eggs, while others are active against neanides or adult mites.

The known acaricides belong to different chemical classes such as organo-phosphoric compounds, haloaromatics, carbamates, metal-organic compounds, etc.

The main known acaricide compounds have been collected by J. C. Street in "Basis for Selectivity of Acaricides" Chap. VIII, page 155, "Pesticide Selectivity" Dekker Inc.-New York (1975).

Recently, acaricide compounds belonging to the class of the alkoxy-diphenyl-ethers (US―A―4 061 683) of the cyclopropane-carboxylates (US―A―3 995 054) and of the di-propargyl-ethers of glycols having 8 to 12 carbon atoms (GB-A-1 472 839) have been described.

US―A―4 061 683 discloses the compound 1,5-dimethylhexyloxybenzyl-propargylether. Said compound shows no activity at all in a test of the inhibitory effects against different insects. US-A-3 908 000 discloses in its abstract propynyl benzyl ethers which are (lower)alkoxy substituted and which have juvenile hormone-like activity. FR-A-2 083 684 discloses insecticidal and acaricidal mixtures. One component may be the 1,5-dimethylhexyloxybenzyl propargylether, a compound with juvenile hormone-like activity.

In spite of the great variety of known acaricide compounds, the mites are still a serious problem due to the damages they cause to the plants, since they reproduce in more generations during one season, thus facilitating the occurrence of phenomena of resistance to this acaricide products utilized.

We have now found new compounds, which are the object of this invention, having general formula: 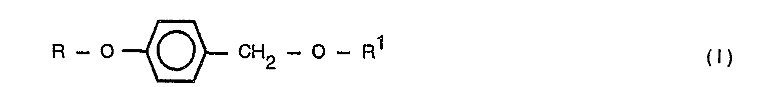 in which:
a substituent selected from R and R¹ represents an alkyl C₇―C₁₁ while the other represents a group 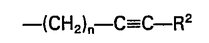 wherein:
   n is an integer from 1 to 4 and
   R² is a hydrogen atom or a group 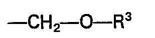
   R³ is a hydrogen atom, an alkyl C₁-C₄, an alkenyl C₂-C₄ or a group 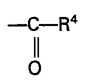 and
   R⁴ is an alkyl C₁―C₄, a cycloalkyl C₃―C₆, a benzyl or phenyl,
with the proviso that when R = alkyl C₇―C₁₁ and R¹ is (CH₂)ₙ-C≡C―H n cannot be 1.

In the above formula the various residues and indices may be defined as follows (in conformity with the above definitions):
R, R¹ = an alkyl group with 7, 8, 9, 10 or 11 carbon atoms, such as heptyl, octyl, i-octyl, nonyl, i-nonyl, decyl, undecyl and dodecyl;
R₃ = e.g. methyl, ethyl, propyl, i-propyl, butyl, i-butyl, sec-butyl, tert-butyl, vinyl or a propenyl or butenyl group;
R₄ = e.g. alkyl as R₃, cyclopropyl, cyclobutyl, cyclopentyl and cyclohexyl.
n = 1,2, 3 or 4.

The compounds of formula I are endowed with a high acaricide activity which is exerted particularly against the eggs of acari.

The synthesis of the compounds of formula I is accomplished according to reactions known in themselves in the usual practice of organic chemistry.

Whenever used in the description of the synthesis processes given hereinbelow, the symbols R, R¹, R², R³, R⁴ and n have the same meanings specified for general formula I, unless otherwise specified.

A suitable starting product for the synthesis is potassium 4-hydroxymethyl-phenate (A) or the 4-hydroxymethyl-phenate of any other alkali metal, easily preparable from 4-hydroxy-benzyl alcohol by treatment with alkali hydroxides or carbonates.

The compounds of formula I, in which R is an alkyl C₇―C₁₁ and R¹ a group ―(CH₂)ₙ―C≡C―R², are obtained according to the reactions' sequence shown in the following Scheme 1 and discussed later on herein.

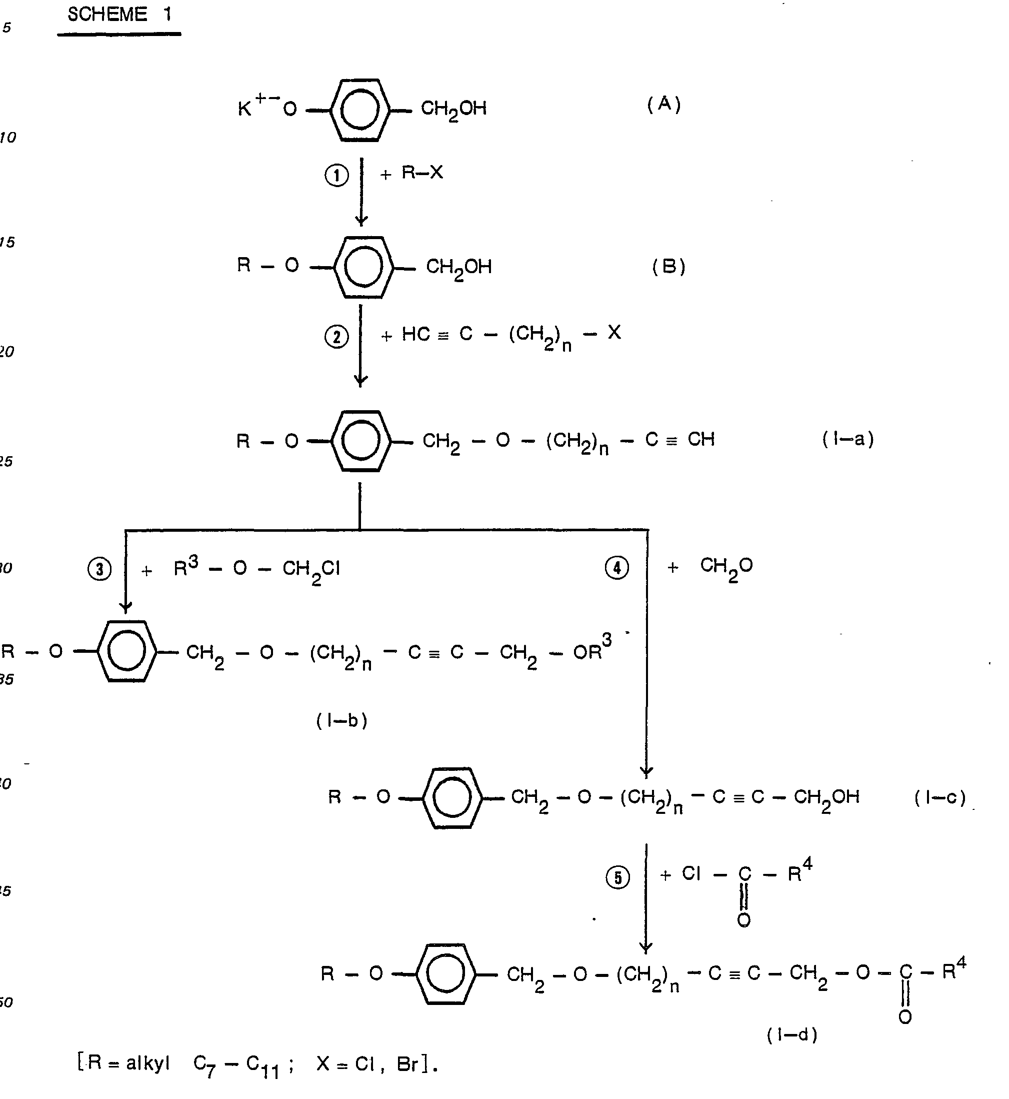

Reaction 1 of Scheme 1 is conducted by condensing the alkali salt (A) with an alkyl halide (R―X) according to conventional techniques.

Intermediate (B) is thus obtained which, in the presence of strong bases (sodium hydride, alkali hydroxides, potassium ter.butylate), reacts with the halides of formula HC≡C-(CH₂)ₙ―X (reaction 2) providing the compounds of formula I in which R = an alkyl C₇―C₁₁ and R² = H (compounds I-a).

The other compounds of formula I, in which R = an alkyl C₇―C₁₁, are prepared by salifying compounds I-a on the triple terminal bond by treatment with alkyl-magnesium bromide or with lithium butyl.

The salts so obtained, by reaction with chloromethyl-alkyl-ethers or chloromethyl-alkenyl-ethers (reaction 3), provide the compounds of formula I in which R = an alkyl C₇―C₁₁, R² =―CH₂OR³ and R³ = an alkyl or alkenyl (I-b).

The reaction of the aforesaid salts with formaldehyde (reaction 4) provides the compounds of formula I in which R = an alkyl C₇―C₁₁, R² = ―CH₂―OR³ and R³ = H (I-c).

The latter provide, by reaction with suitable acyl chlorides (reaction 5), the compounds of formula I in which R = an alkyl C₇―C₁₁, R² = ―CH₂OR³ and R³ = 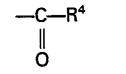 (I-d).

The synthesis of the compounds of formula I, in which R is a group ―(CH₂)ₙ―C≡C―R² and R¹ is an alkyl C₇―C₁₁, is carried out in an analogous manner.

Phenate (A) is condensed with the alkyl halide of formula 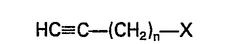 and the benzyl alcohol (B') so obtained is condensed with the alkyl halide 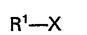 (in which R¹ = an alkyl C₇―C₁₁ and X = Cl, Br).

From these reactions, the compounds of formula 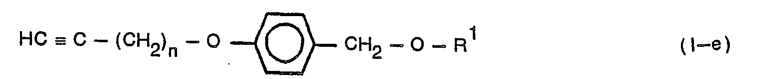 are obtained.

Starting from compounds I-e one proceeds, by employing reactions analogous with reactions 3, 4 and 5 of Scheme 1, to the synthesis of the other derivatives of formula I wherein R¹ is an alkyl C₇―C₁₁.

The reactions are shown in the following Scheme 2.

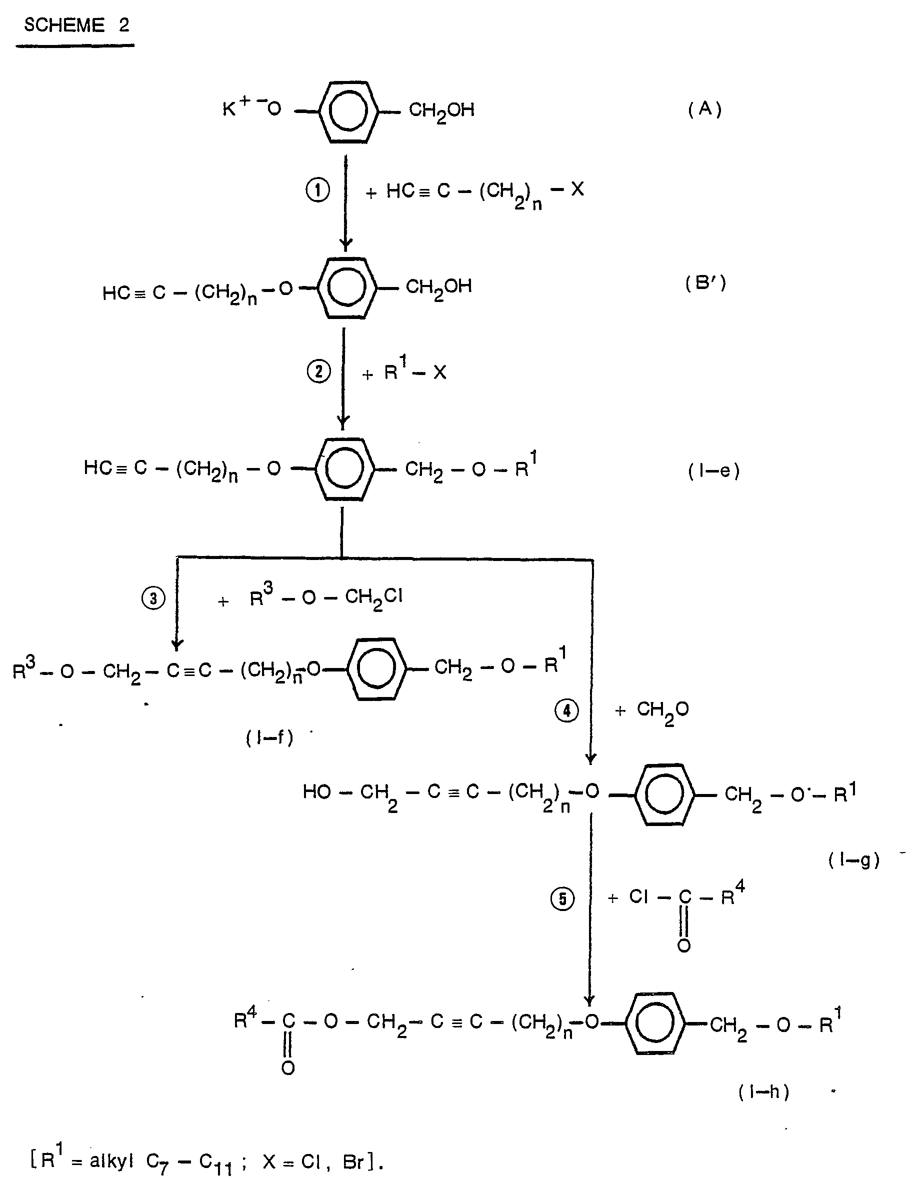

As is mentioned hereinbefore, the compounds of formula I are endowed with a high acaricide activity.

Said compounds are active mainly against the mite eggs and exert simultaneously a cetain activity also against the movable forms of mites. Compounds of formula I are endowed furthermore with an egg-killing activity against the hibernating eggs of mites: therefore they possess a characteristic which is difficult to be found in other acaricide products.

The compounds of formula I are endowed also with a high action persistence, which is a very important characteristic for egg-killing compounds, as well as by a very low toxicity towards warm-blooded animals and fish and are completely harmless towards useful insects such as, for example, bees.

The main mites of particular economic interest due to the damages caused to the plants and due to their extreme spreading in all the cultivated areas, prevailingly belong to family Tetranychidae, genera Tetranychus (T. urticae, T. telarius, T. pacificus, etc), Panonychus (P. ulmi, P. citri, etc.), Bryobia (B. praetiosa) and Oligonychus.

Other noxious species for the cultures are present, for example, in the families Eriophyidae (genera Aceria, Eriophyes, Phyllocoptes, Phyllocoptruta, Vasates, etc.), Tarsonemidae (genus Hemitarsonemus) and Tenuipalpidae.

The acaricide compounds of general formula I can be usefully employed in the fight against the mites both as such and in the form of proper compositions or fomulations.

In the compositions or formulations there may be present, besides one or more compounds of general formula I as active ingredient, suitable vehicles, either solid or liquid, as well as additives, such as surfactants, wetting agents, adhesion-promoting agents and dispersants.

The compounds can be easily formulated as granules, powders, wettable powders, pastes, emulsions, emulsifiable concentrates, solutions, etc., according to techniques well known in the usual formulative practice.

Whenever required by particular purposes, such as for example the necessity of broadening the activity range of the compositions, or particular environmental conditions, it is possible to add other active substances, such as insecticides and/or acaricides, to the compositions and formulations.

The active substances suited to be co-formulated include phosphorus-organic compounds, pyrethroids, nitrophenols, formamidines, urea derivatives, carbamates, chlorinated hydrocarbons, organo- metal compounds and mineral oils.

In the above-mentioned compositions the active substance may be contained in amounts ranging from 0.5 to 90% by weight, depending on the composition type and on the particular treatment in which it is employed. The amount of composition to be distributed in the area to be protected from mite infestations depends on various factors, such as for example the type of composition or formulation, the type of application, the nature and degree of the infestation, the kind of culture to be protected, climatic and environmental conditions.

The following examples are given to better illustrate the present invention.
General remark: the nuclear magnetic resonance spectra ('H-NMR) indicated in the examples have been recorded by using CDCI₃ as a solvent and TMS as an internal standard.

In the description of the spectra, the following abbreviations are used: s = singlet, d = doublet, t = triplet, m = multiplet or unresolved complex signal.

### Example 1

### Preparation of 4-decyloxy.benzyl alcohol [intermediate 1]

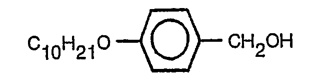

### (Reaction 1, Scheme 1)

To a solution of 12 g of 4-hydroxy-benzyl alcohol in 60 ml of dimethylsulphoxide (DMSO), 7 g of ground KOH were added. The mixture was stirred for 30 minutes at room temperature, then it was cooled down to 10°C, and 22 g of 1-bromo-decane were added thereto.

The reaction mixture was maintained under stirring at room temperature for 6 hours, then it was poured into water and extracted with ethyl ether (Et_{z}O).

The ethereal phases were joined and then washed with aqueous soda at 5% and with water until a neutral pH was attained. After anhydrification, the solvent was removed by evaporation at reduced pressure.
the assigned structure).

### Example 2

### Preparation of 4-decyloxy-benzyl-propargylether (intermediate 2]

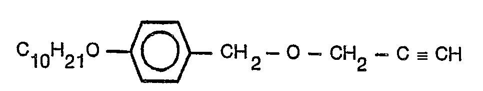

### (Reaction 2, Scheme 1)

2.6 g of intermediate 1 (see example 1) were added to 40 ml of dimethylformamide (DMF) containing 0.7 g of ground KOH. The mixture was stirred at room temperature for 30 minutes, then it was cooled to 0°C, and a solution of propargyl bromide (2 g) in 5 ml of DMF was added thereto.

The reaction mixture was stirred for 6 hours at room temperature and was then poured into water and extracted with Et₂0.

The joined ethereal phases were washed with water to neutral pH and anhydrified, and the solvent was evaporated at reduced pressure.

The residue was subjected to chromatography on silica gel (eluent: hexane - Et₂0 in a ratio of 9: 1). 2.3 g of the desired product were so obtained.
NMR (5, ppm)
0.85 (t, 2H)
1-2 (m, 17H)
3.85 (t, 2H)
4.1 (t, 2H)
4.45 (s, 2H)
6.75―7.15 (d, d, 4H).

### Example 3

### Preparation of 4-decyloxy-benzyl-(hexa-5-oxa-2-inyl)-ether [Compound No.1]

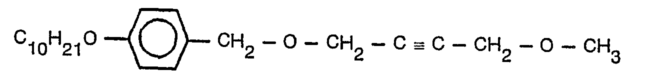

### (Reaction 3, Scheme 1)

1 g of intermediate No. 2 (see example 2) was added dropwise to a solution of 0.5 moles of C₂H₅MgBr in 10 ml of anhydrous tetrahydrofuran (THF) kept in a nitrogen atmosphere. On completion of the addition the mixture was gradually heated up to 60°C and then was allowed to cool at room temperature.

To such solution cooled down to -5-0°C, a soluton of 5.10-³ moles of chloromethyl-methyl-ether (CH₃0CH₂CI) in 5 ml of anhydrous THF was added dropwise.

The temperature was allowed to rise up to the room temperature value and after 1 hour stirring the mixture was poured into a saturated solution of NH₄CI.

Successively it was extracted with Et₂0 and the joined ethereal extracts were anhydrified and the solvent was evaporated under reduced pressure.

The rough product was purified by chromatography on a silica gel column (eluent: hexane-Et₂0 in the ratio 95:5), thus obtaining 0.5 g of the desired product.
NMR (δ, ppm)
0.85 (t, 3H)
1-2 (m, 16H)
3.4 (s, 3H)
3.75 (t, 2H)
4.1 (s, 4H)
4.4 (s, 2H)
6.75-7.15 (d, d, 4H).

### Example 4

### Preparation of 4-decyloxy-benzyl-pent-4-ynyl-ether [Compound No. 2]

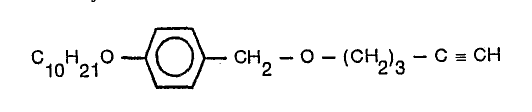

### (Reaction 2, Scheme 1)

2.6 g of intermediate 1 (see example 1) were added to 40 ml of dimethylformamide (DMF) containing 0.7 g of ground KOH. The mixture was stirred at room temperature for 30 minutes, then it was cooled to 0°C, and a solution of propargyl bromide (2 g) in 5 ml of DMF was added thereto.

The reaction mixture was stirred for 6 hours at room temperature and was then poured into water and extracted with Et₂0.

The joined ethereal phases were washed with water to neutral pH and anhydrified, and the solvent was evaporated at reduced pressure.

The residue was subjected to chromatography on silica gel (eluent: hexane - Et₂0 in a ratio of 9: 1).

2.3 g of the desired product were thus obtained.
NMR (δ, ppm)
0.85 (t, 3H)
1-2.2 (m, 19H)
2.25 (m, 2H)
3.5 (t, 2H)
3.9 (t, 2H)
4.45 (s, 2H)
6.7-7.15 (d,d,4H)

I.R.: meaningful bands at 3300 and 2150 cm-¹.

### Example 5

### Preparation of 4-decyloxy-benzyl-(6-hydroxy-hex-4-ynyl)ether [Compound No. 3]

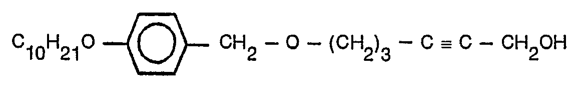

### (Reaction 4, Scheme 1)

4 ml of a solution of butyl-lithium at 15% in hexane were added to a solution of 2.2 g of Compound No. 3 (see example 4) in 50 ml of anhydrous THF maintained at -10°C.

The mixture was stirred for 30 minutes, whereupon 0.2 g of paraformaldehyde were added thereto. The reaction mixture was then stirred for 1 hour at 40°C. After cooling, it was poured into water and it was extracted with Et₂0.

The joined ethereal phases were anhydrified and the solvent was removed by evaporation under reduced pressure.

The residue was purified by chromatography on a silica gel column (eluent: hexane―Et₂O in a ratio of 1:1).

2 g of the desired product were thus obtained.
NMR (δ, ppm)
0.85 (t, 3H)
1-2 (m, 18H)
2.3 (m, 3H)
3.5 (t, 2H)
3.9 (t, 2H)
4.0 (s, 2H)
4.45 (s, 2H)
6.7-7.15 (d, d, 4H)

### Example 6

### Preparation of 8-(4-decyloxyphenyl)-7-oxa-2-octynyl acetate (Compound No. 4]

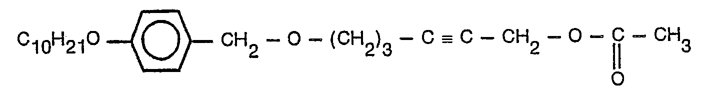

### (Reaction 5, Scheme 1)

1 g of Compound No. 3 (see example 5) was dissolved in 20 ml of benzene containing 1 ml of pyridine.

0.5 g of acetyl chloride were then added under stirring. After stirring for 1 hour at room temperature, the reaction mixture was poured into water and extracted with Et₂0.

The joined ethereal phases were anhydrified and the solvent was removed by evaporation under reduced pressure.

1 g of the desired product was thus obtained.
NMR (δ, ppm)
0.85 (t, 3H)
1-2 (m, 18H)
2 (s, 3H)
2.3 (m, 2H)
3.5 (t, 2H)
3.9 (t, 2H)
4.0 (s, 2H)
4.45 (s, 2H)
6.7-7.15 (d, d, 4H).

### Example 7

### Preparation of 4-decyloxy-benzyl-(7-oxa-4-octynyl)-ether [Compound No. 5].

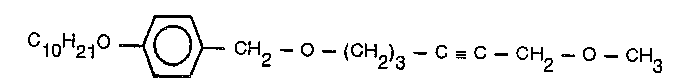

### (Reaction 3, Scheme 1).

4 ml of a solution at 15% of butyl-lithium in hexane were added dropwise to a solution of 2 g of Compound No. 2 (see example 4) in 20 ml of anhydrous THF maintained at -10°C in a nitrogen atmosphere.

The mixture was cooled down to -40°C, whereupon 0.5 ml of CH₃0CH₂CI dissolved in 6 ml of anhydrous THF were added dropwise thereinto.

The temperature was allowed to gradually rise to 0°C

The reaction mixture was then poured into a saturated NH₄CI solution. The organic layer was separated and the aqueous solution was extracted with Et₂0.

The joined organic phases were anhydrified and the solvent was removed under reduced pressure.

The raw product was purified by chromatography on silica gel (eluent: hexane-Et₂0 in a ratio of 95:5).

1.5 g of the desired product were thus obtained.
NMR (δ, ppm)
0.85 (t, 3H)
1-2 (m, 18H)
2.25 (m, 2H)
3.3 (s, 3H)
3.5 (t, 2H)
3.85 (t, 2H)
4 (s, 2H)
4.35 (s, 2H)
6.75-7.15 (d, d, 4H).

### Example 8

### Preparation of 4-propargyloxy-benzyl alcohol [intermediate 3]

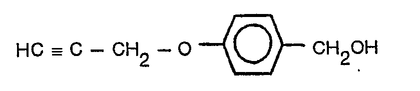

### (Reaction 1, Scheme 2)

7 g of potassium carbonate were dispersed in a solution of 6 g of 4-hydroxy-benzyl alcohol in 40 ml of DMSO.

6 g of propargyl bromide were added dropwise to the mixture.

The reaction mixture was stirred for 6 hours at room temperature and was then poured into water and extracted with Et₂0.

The joined ethereal phases were anhydrified and the solvent was removed by evaporation under reduced pressure.

The residue was subjected to chromatography on silica gel (eluent: hexane-Et₂0 in a ratio of 3:1), thus obtaining 8 g of the desired product.
NMR (δ, ppm)
2.3 (d, 1H)
2.5 (s, 1H)
4.4 (s, 2H)
4.5 (d, 2H)
6.8-7 (d, d, 4H).

### Example 9

### Preparation of 4-propargyloxy-benzyl-decyl-ether [Compound No. 6]

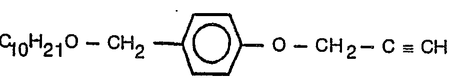

### (Reaction 2, Scheme 2).

A solution of 3.2 g of intermediate No. 3 (see example 8) in 5 ml of DMSO was added dropwise to a dispersion of 10 g of NaH at 50% in paraffin and 25 ml of DMSO. The mixture was kept under stirring for 30 minutes, whereafter a solution of 4.4 g of 1-bromo-decane in 5 ml of DMSO was added to such mixture.

The reaction mixture was heated, under stirring, at 45°C for 3 hours. After cooling, the mixture was poured into water and extracted with Et₂0.

The joined ethereal phases were anhydrified and the solvent was removed by evaporation under reduced pressure.

The residue (5 g) was purified by chromatography on a silica gel column (eluent: hexane-Et₂0 in a ratio of 9:1).

4.3 g of the desired product were thus obtained.
NMR (δ, ppm)
0.85 (t, 3H)
1-2 (m, 16H)
2.3 (d, 1H)
3.4 (t, 2H)
4.4 (s, 2H)
4.5 (d; 2H)
6.8-7.2 (d, d, 4H)

### Example 10

### Preparation of4-[(hex-5-oxa-2-ynyl)-oxy]-benzyl-decylether [Compound No. 7]

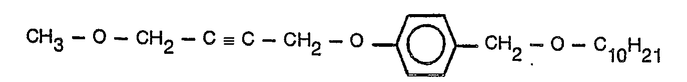

### (Reaction 3, Scheme 2).

6.6 ml of a solution at 15% of butyl-lithium in hexane were added to a solution of 3 g of Compound No. 6 (see example 9) in 30 ml of anhydrous THF maintained at -10°C.

The mixture was then cooled down to -40°C and a solution of 0.8 g of CH₃OCH₂CI in 10 ml of anhydrous THF was then added dropwise to said mixture.

The temperature was allowed to gradually rise to 0°C while going on stirring, and the mixture was then poured into a saturated NH₄CI solution.

The organic layer was separated and the aqueous phase was extracted with Et₂0.

The joined organic phases were anhydrified and the solvent was removed by evaporation under reduced pressure.

The residue was subjected to chromatography on a silica gel column (eluent: hexane-Et₂0 in a ratio of 95:5).

2.2 g of the desired product were thus obtained.
NMR (δ, ppm)
0.85 (t, 3H)
1-2 (m, 16H)
3.35 (s, 3H)
3.4 (t, 2H)
4.1 (s, 4H)
4.45 (s, 2H)
6.65-7.15 (d, d, 4H)

### Example 11

### Preparation of 4-(pent-4-ynyloxy)-benzyl alcohol [intermediate No. 4]

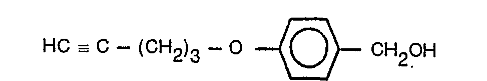

### (Reaction 1, Scheme 2).

0.6 g of ground NaOH were added to a solution of 1.8 g of 4-hydroxy-benzyl alcohol in 25 ml of DMSO.

After 30 minutes, a solution of 2.3 g of 4-pentynyl chloride in 5 ml of DMSO was added to said mixture.

The mixture was stirred at 60°C for 3 hours.

After cooling it was poured into water and it was extracted with Et₂O.

The joined ethereal phases were anhydrified and the solvent was removed by evaporation under reduced pressure. The residue was purified by chromatography on a silica gel column (eluent: hexane-Et₂0 in.a ratio of 3:1). 2.3 g of the desired product were thus obtained.
NMR (δ, ppm)
1.8―2.6 (m, 6H)
4.0 (t, 2H)
4.4 (s, 2H)
6.65-7.15 (d, d, 4H).

### Example 12

### Preparation of [4-(pent-4-ynyloxy)-benzyl]-decyl-ether [Compound No. 8]

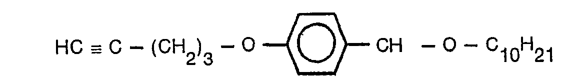

### (Reaction 2, Scheme 2).

A solution of 1.9 g of intermediate No. 4 (see example 11) in 5 ml of DMSO was added to a dispersion of 0.5 g of NaH at 50% in paraffin in 5 ml of DMSO kept under stirring in a nitrogen atmosphere at room temperature.

After 1 hour, 1.8 ml of 1-bromo-decane were added by dropping. After a beginning of exothermy, the reaction mixture was stirred at 50°C for 3 hours.

After cooling, the mixture was poured into water and extracted with Et₂0.

The joined ethereal phases were anhydrified and the solvent was removed by evaporation at reduced pressure.

The residue was subjected to chromatography on a silica gel column (eluent: hexane-Et₂0 in a ratio of 9:1). 2.4 g of the desired product were thus obtained.
NMR (δ, ppm)
0.85 (t, 3H)
1―2.6(m,21H)
3.35 (t, 2H)
4.0 (t, 2H)
4.4 (s, 2H)
6.65-7.15 (d, d, 4H).

### Example 13

### Preparation of 4-[(oct-7-oxa-4-ynyl)oxy]-benzyl-decylether [Compound No.9]

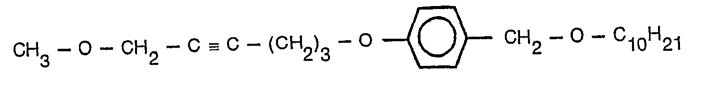

### (Reaction 3, Scheme 2).

To a solution of 3.3 g of Compound No. 8 (see example 12) in 20 ml of anhydrous THF maintained under stirring in a nitrogen atmosphere at -10°C there were added 6.5 ml of 15% butyl-lithium in hexane.

After 30 minutes the mixture was cooled down to -40°C and 0.8 g of CH₃-O-CH_{Z}CI dissolved in 10 ml of anhydrous THF were added thereto.

Temperature was allowed to gradually rise to 0°C and the mixture was poured into a saturated NH₄CI solution.

The organic phase was separated and the aqueous phase was extracted with Et₂0. The joined organic phases were anhydrified and the solvent was removed by evaporation at reduced pressure.

The residue was subjected to chromatography on a silica gel column (eluent: hexane-Et₂0 in a ratio of 95:5). 2.8 g of the desired product were thus obtained.
NMR (6, ppm)
0.85 (t, 3H)
1-2.6 (m, 20H)
3.3 (s, 3H)
3.35 (t, 2H)
4.0 (t, 2H)
4.05 (s, 2H)
4.35 (s, 2H)
6.65-7.15 (d, d, 4H).

### Example 14

### Determination of the acaricide activity.

To evaluate the acaricide activity, the mites belonging to the family Tetranychidae were taken into particular consideration, such mites being recognized as representative and significant due to the importance of the damages caused to the cultures and due to their diffusion.

### Determination of the activity against Tetranychus urticae (eggs).

Small discs cut from bean leaves were infested with eggs of said mite and successively sprayed with a hydroacetonic solution of the product being tested.

The percentage of unhatched eggs, which corresponds to the percentage of mortality, was evaluated 7 days after the treatment by comparing it with the one of eggs treated only with a hydroacetonic mixture.

### Determination of the activity against Tetranychus u. adults.

Small discs cut from bean leaves were infested with adult acari and successively treated by spraying thereonto a hydroacetonic dispersion of the product being tested.

The percentage of mortality was evaluated 24 hours after the treatment by comparison with the one of acari treated only with a hydroacetonic mixture.

### Determination of the activity on Panonychus ulmi (eggs).

Small discs cut from apple-tree leaves were infested with eggs of said acarus and were successively treated by spraying thereonto a hydroacetonic solution of the product being tested.

The percentage of unhatched eggs was evaluated 10 days after the treatment by comparison with the one of eggs treated only with a hydroacetonic mixture.

On following Table 1 there are recorded the data relating to the acaricide activity, at the indicated doses, of compounds according to the present invention, expressed conforming to the following scale of values:
4: full activity, mortality exceeding 90%
3: high activity, mortality ranging from 60 to 90%
2: sufficient activity, mortality ranging from 40 to 60%,
1: poor activity, mortality ranging from 10 to 40%,
0: negligible activity or no activity, mortality ranging from 0 to 10%.

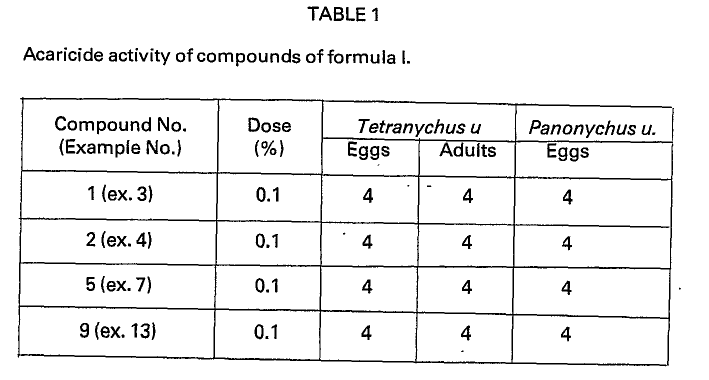

## Claims

1. Compounds of general formula: 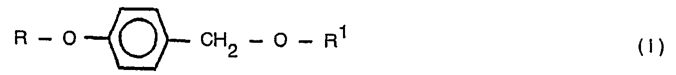 in which:
one of the substituents R and R¹ represents an alkyl C₇―C₁₁ and the other represents a group 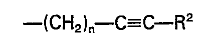 wherein:
n is an integer from 1 to 4 and
R² is a hydrogen atom or a group 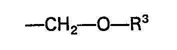
R³ is a hydrogen atom, an alkyl C₁-C₄, an alkenyl C₂―C₄ or a group 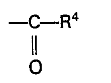 and
R⁴ is an alkyl C₁-C₄, a cycloalkyl C₃―C₆, a benzyl or phenyl,
with the proviso that when R = alkyl C₇―C₁₁ and R¹ is (CH₂)ₙ―C≡C―H n cannot be 1.

2. Compounds according to claim 1, in which R represents an alkyl C₇―C₁₁ and R¹ a group ―(CH₂)ₙ―C≡C―R².

3. Compounds according to claim 2, in which n = 1 or 3.

4. Compounds according to claim 3, in which R² =―CH₂―O―R³ and R³ = H, CH₃,―CO―CH₃.

5. Compounds according to claim 1, in which R represents a group 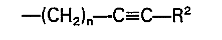 and R¹ represents an alkyl C₇―C₁₁.

6. Compounds according to claim 5, in which n = 1 or 3.

7. Compounds according to claim 6, in which R² = H.

8. Compounds according to claim 6, in which R² = ―CH₂―O―R³ and R³ = H, CH₃, ―COCH₃.

9. A process for preparing the compounds of formula I in which R = an alkyl C₇―C₁₁, R¹ is ―(CH₂)ₙ―C≡C―R², R² = H, and n is an integer from 2 to 4, characterized in that an alkali phenate of 4-hydroxy-benzyl alcohol is reacted with an alkyl halide having from 7 to 11 carbon atoms and the resulting product is treated with strong bases and condensed with a compound of formula 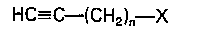 (in which X=CI, Br).

10. A process for preparing the compounds of formula I in which R =―(CH₂)ₙ―C≡C―R², R² = H and R¹ = an alkyl C₇―C₁₁, characterized in that an alkali phenate of 4-hydroxy-benzyl alcohol is reacted with a compound of formula 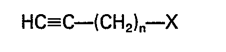 (in which X = Cl, Br)
and the resulting product is treated with strong bases and condensed with an alkyl halide having from 7 to 11 carbon atoms.

11. A process for preparing the compounds of formula I, in which R² = ―CH₂―OR³, characterized in that a compound of formula 1, in which R² = H, is treated with strong bases to salify the carbon atom of the triple terminal bond and the resulting salt is reacted with chloromethyl-alkyl-ether or chloromethyl-alkenyl- ether in order to obtain the compounds of formula I in which R³ is an alkyl or an alkenyl; with formaldehyde to obtain the compounds in which R³ = H and these latter compounds, by reaction with acyl chlorides of formula 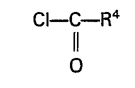 provide the compounds offormula I in which R³ =

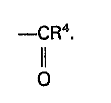

12. A method of fighting infestations of mites on useful plants, consisting in distributing on the plants as effective amount of one or more compounds of claim 1 to 8, either as such or in the form of suitable compositions.

13. Acaricide compositions containing as active ingredient one or more of the compounds of claim 1 to 8 along with inert vehicles and, optionally, other additives.

## Patentansprüche

1. Verbindungen der allgemeinen Formel 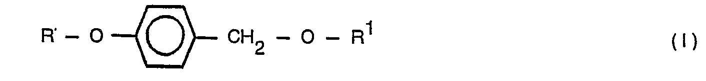 in welcher
einer der Substituenten R und R¹ eine C₇―C₁₁ Alkylgruppe und der andere eine Gruppe ―(CH₂)ₙ―C≡C―R² bedeutet, worin n eine ganze Zahl von 1 bis 4 ist und
R² ein Wasserstoffatom oder eine Gruppe ―CH₂―O―R³ bedeutet,
R³ ein Wasserstoffatom, eine C₁―C₄ Alkylgruppe, eine C₂―C₄ Alkenylgruppe oder eine Gruppe 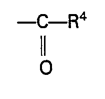 bedeutet und R⁴ eine C₁-C₄ Alkylgruppe, eine C₃-C₆ Cycloalkylgruppe, eine Benzyl oder eine Phenylgruppe bedeutet, mit der Bedingung, daß-wenn R = C₇―C₁₁ Alkyl ist und R₁ (CH₂)ₙC≡C―H ist―n nicht 1 sein kann.

2. Verbindungen nach Anspruch 1, in welchen R für C₇―C₁₁ Alkyl steht und R¹ eine Gruppe -(CH₂)ₙC≡C-R² ist.

3. Verbindungen nach Anspruch 2, in welchen n 1 oder 3 ist.

4. Verbindungen nach Anspruch 3, in welchen R² ―CH₂-O-R³ ist und R³ H, CH₃ oder ―CO―CH₃ bedeutet.

5. Verbindungen nach Anspruch 1, in welchen R eine Gruppe―(CH₂)ₙC≡C―R² bedeutet und R¹ für eine C₇―C₁₁ Alkylgruppe steht.

6. Verbindungen nach Anspruch 5, in welchen n 1 oder 3 ist.

7. Verbindungen nach Anspruch 6, in welchen R² H ist.

8. Verbindungen nach Anspruch 6, in welchen R² -CH₂-O-R³ ist und R³ H, CH₃ oder -COCH₃ bedeutet.

9. Verfahren zur Herstellung der Verbindungen der Formel (I), in welcher R C₇―C₁₁ Alkyl ist, R¹ ―(eH₂)ₙ―C≡C―R² ist, R² H ist und n eine ganze Zahl von 2 bis 4 ist, dadurch gekennzeichnet, daß man ein Alkaliphenat des 4-Hydroxybenzylalkohols mit einem Alkylhalogenid mit 7 bis 11 Kohlenstoffatomen umsetzt und das erhaltene Produkt mit starken Basen behandelt und mit einer Verbindung der Formel 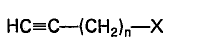 (in welcher X für CI oder Br steht) kondensiert.

10. Verfahren zur Herstellung der Verbindungen der Formel (I), in welcher R für ―(CH₂)ₙ―C≡C―R² steht, R² H bedeutet und R¹ C₇―C₁₁ Alkyl bedeutet, dadurch gekennzeichnet, daß man ein Alkaliphenat des 4-Hydroxybenzylalkohols mit einer Verbindung der Formel 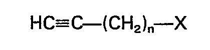 (in welcher X für CI oder Br steht) umsetzt, und das erhaltene Produkt mit starken Basen behandelt und mit einem Alkylhalogenid mit 7 bis 11 kohlenstoffatomen kondensiert.

11. Verfahren zur Herstellung der Verbindungen der Formel (I), in welcher R² für ―CH₂―OR³ steht, dadurch gekennzeichnet, daß man eine Verbindung der Formel (1), in welcher R² für Hg steht, mit starken Basen behandelt, um das Kohlenstoffatom der dreifachen endständigen Bindung in ein Anion überzuführen, und das erhaltene Salz mit Chlormethylalkylether oder Chlormethylalkenylether zur Bildung der Verbindungen der Formel (I), in welcher R³ Alkyl oder Alkenyl ist, umsetzt oder mit Formaldehyd zur Bildung der Verbindungen, in welchen R³ H bedeutet, umsetzt und diese letztgenannten Verbindungen durch Reaktion mit Acylchloriden der Formel 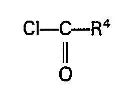 die Verbindungen der Formel (1), in welcher R³ für 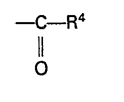 steht, ergeben.

12. Verfahren zur Bekämpfung von Milbenbefall auf Nutzpflanzen, bestehend in der Verteilung einer wirksamen Menge einer oder mehrerer der Verbindungen von Anspruch 1 bis 8 als solche oder in Form geeigneter Zusammensetzungen auf die Pflanzen.

13. Acarizide Zusammensetzung, enthaltend als aktiven Bestandteil eine oder mehrere Verbindungen von Anspruch 1 bis 8 zusammen mit inerten Trägern und wahlweise anderen Zusätzen.

## Revendications

1. Composés de formule générale: 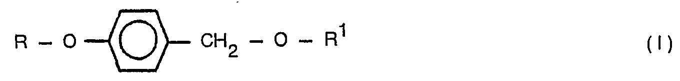 dans laquelle un des substituants R et R¹ représente un radical alkyle comprenant 7 à 11 atomes alors que l'autre présente un groupe: 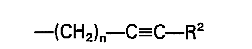 dans laquelle:
n est un nombre entier compris entre 1 et 4; et
R² est un atome d'hydrogène ou un groupe: 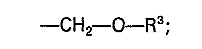
R³ est un atome d'hydrogène, un radical alkyle comprenant 1 à 4 atomes de carbone, alkényle comprenant 2 à 4 atomes de carbone ou un group: 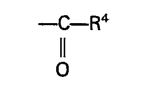 et
R⁴ est un radical alkyle comprenant 1 à 4 atomes de carbone, cycloalkyle comprenant 3 à 6 atomes de carbone, benzyle ou phényle;
étant entendu que lorsque R est un radical alkyle comprenant 7 à 11 atomes de carbone et que R¹ est un groupe (CH₂)ₙ―C≡C―H, n ne peut pas être égal à 1.

2. Composés selon la revendication 1, dans laquelle R représente un radical alkyle comprenant 7 à 11 atomes de carbone et R¹ est un groupe (CH₂)ₙ―C≡C―R².

3. Composés selon la revendication 2, dans laquelle n est égal à 1 ou 3.

4. Composés selon la revendication 3, dans laquelle R² =―CH₂―O―R³ et R³ = H, CH₃, ―CO―CH₃.

5. Composés selon la revendication 1, dans laquelle R représente un groupe 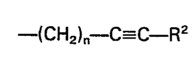 et R¹ représente un radical alkyle comprenant de 7 à 11 atomes de carbone.

6. Composés selon la revendication 5, dans laquelle n est égal 1 ou 3.

7. Composés selon la revendication 6, dans laquelle R² = H.

8. Composés selon la revendication 6, dans laquelle R² = ―CH₂―O―R³ et R³ = H, CH₂,―CO―CH₃.

9. Un procédé de préparation des composés de formule (I), dans laquelle:
R est un radical alkyle comprenant 7 à 11 atomes de carbone;
R¹ est un groupe (CH₂)ₙ―C≡C―R²
_{R}² égal H, et
n est un nombre entier compris entre 2 et 4,
caractérisé en ce qu'un phénate alcalin de l'alcool 4-hydroxy-benzylique est amené à réagir avec un halogénure d'alkyle comprenant 7 à 11 atomes de carbone puis le produit résultant est traité par des bases fortes et condensé avec un composé de formule: 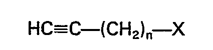 (dans laquelle X = CI, Br).

10. Un procédé de préparation des composés de fomule (1), dans laquelle:
R = ―(CH₂)ₙ―C≡C―R²
R²=H,et
R¹ = un radical alkyle comprenant 7 à 11 atoms de carbone,
caractérisé en ce qu'un phénate alcalin de l'alcool 4-hydroxy-benzylique est amené à réagir avec un composé de formule: 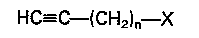 (dans laquelle X = CI, Br)
et en ce que le produit résultant est traité par des bases fortes puis condensé avec un halogénure d'alkyle comprenant 7 à 11 atomes de carbone.

11. Un procédé de préparation des composés de formule (I), dans laquelle R² = ―CH₂―OR³, caractérisé en ce qu'un composé de formule (I) dans laquelle R² = H est traité par des bases fortes pour salifier i'atome de carbone de la triple liaison terminale et en ce que le sel résultant est amené à réagir avec un chlorométhyl-alkyl-éther ou un chlorométhyl-alkényl-éther afin d'obtenir des composés de formule (I) dans laquelle R³ est un radical alkyle ou alkényle, avec un formaldéhyde pour obtenir des composés dans lesquels R³ = H et ces derniers composés, par réaction avec des chlorures d'acyles de formule: 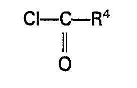 conduisent aux composés de formule (I) dans laquelle R³ = ―C―O―R⁴.

12. Un procédé pour lutter contre les infestations dues aux mites vis-à-vis des plantes utiles consistant à répartir sur les plantes une quantité efficace d'un ou plusieurs des composés selon les revendications 1 à 8, soit tels quels, soit sous la forme d'une composition convenable.

13. Composition acaricide contenant en tant qu'ingrédient actif un ou plusieurs des composés selon les revendications 1 à 8 associés à des véhicules inertes et, éventuellement, à d'autres additifs.
